# EUROPEAN PATENT SPECIFICATION

(11) **EP 1 367 130 B1**
(45) Date of publication and mention of the grant of the patent: **21.03.2007**
(21) Application number: 03010869.0
(22) Date of filing: 15.05.2003
(51) Int. Cl.: C12P 13/08

(54) **Process for the preparation of L-lysine using coryneform bacteria which contain an attenuated malate enzyme gene**
Verfahren zur Herstellung von L-Lysin unter Verwendung Coryneformer Bakterien die ein attenuiertes Malat Enzym-gen enthalten
Procédé pour la préparation de L-Lysine au moyen de bactéries coryneformes qui contiennent un malate-enzyme gène attenué

(30) Priority: 31.05.2002 DE 10224088
(43) Date of publication of application: 03.12.2003
(73) Proprietor: Degussa GmbH, 40474 Düsseldorf (DE)
(72) Inventor: Bathe, Brigitte, Dr., 33154 Salzkotten (DE); Farwick, Mike, Dr., 33615 Bielefeld (DE); Pfefferle, Walter, Dr., 33790 Halle (DE); Marx, Achim, Dr., 97404 Banska Bystrica (SK)

(56) References cited:
- FR-A- 2 796 080
- GOURDON P ET AL: "Cloning of the malic enzyme gene from Corynebacterium glutamicum and role of the enzyme in lactate metabolism." APPLIED AND ENVIRONMENTAL MICROBIOLOGY, vol. 66, no. 7, July 2000 (2000-07), pages 2981-2987, XP002252045 US ISSN: 0099-2240
- RIEDEL C ET AL: "CHARACTERIZATION OF THE PHOSPHOENOLPYRUVATE CARBOXYKINASE GENE FROM CORYNEBACTERIUM GLUTAMICUM AND SIGNIFICANCE OF THE ENZYME FOR GROWTH AND AMINO ACID PRODUCTION" JOURNAL OF MOLECULAR MICROBIOLOGY AND BIOTECHNOLOGY, HORIZON SCIENTIFIC PRESS, WYMONDHAM,, GB, vol. 3, no. 4, 2001, pages 573-583, XP001031161 ISSN: 1464-1801
- SCHRUMPF B ET AL: "A FUNCTIONALLY SPLIT PATHWAY FOR LYSINE SYNTHESIS IN CORYNEBACTERIUM GLUTAMICUM" JOURNAL OF BACTERIOLOGY, WASHINGTON, DC, US, vol. 173, no. 14, July 1991 (1991-07), pages 4510-4516, XP000884180 ISSN: 0021-9193

## Description

### Field of the Invention

The invention relates to a process for the preparation of L-lysine using coryneform bacteria in which the mez gene, which codes for the malate enzyme (malic enzyme, EC:1.1.1.40), is attenuated.

### Background of the Invention

L-Amino acids, in particular L-lysine and L-methionine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites, such as, for example, the lysine analogue S-(2-aminoethyl)-cysteine, or are auxotrophic for metabolites of regulatory importance and produce L- amino acids are obtained in this manner.

Methods of the recombinant DNA technique have also been employed for some years for improving the strain of Corynebacterium glutamicum strains which produce L-amino acids, by amplifying individual amino acid biosynthesis genes and investigating the effect on the L-amino acid production.

### Object of the Invention

The inventors had the object of providing new fundamentals for improved processes for the fermentative preparation of L-lysine with coryneform bacteria.

### Summary of the Invention

When L-lysine or lysine are mentioned in the following, not only the bases but also the salts, such as e.g. lysine monohydrochloride or lysine sulfate, are meant by this. The invention relates to a process for the fermentative preparation of L-lysine using coryneform bacteria in which at least the nucleotide sequence which codes for the malate enzyme (malic enzyme) (mez gene) is eliminated.

The malate enzyme catalyses oxidative decarboxylation of malate to pyruvate, a molecule of carbon dioxide being split off.

This invention provides a process for the preparation of L-lysine by fermentation of a recombinant coryneform bacteria, wherein in the bacteria the expression of the nucleotide sequence of Corynebacterium glutamicum which codes for the malate enzyme is eliminated.

This invention also provides a process further comprising the following steps:
a) concentration of the L-lysine in the medium or in the cells of the bacteria; and
b) isolation of the l-Lysine constituents of the fermentation broth and/or the biomass optionally remaining in portions or in their total amounts in the end product.

The strains employed preferably already produce L-lysine before attenuation of the mez gene.

Preferred embodiments are to be found in the claims.

### Detailed Description of the Invention

The term "attenuation" or "attenuate" in this connection describes the reduction or elimination of the intracellular activity or concentration of one or more enzymes or proteins in a microorganism which are coded by the corresponding DNA, for example by using a weak promoter or using a gene or allele which codes for a corresponding enzyme with a low activity or inactivates the corresponding gene or enzyme or protein and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

The microorganisms used by the present invention can prepare amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum, are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
and L-lysine producing mutants or strains prepared therefrom, such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 and
Corynebacterium glutamicum DSM 5715.

It has been found that coryneform bacteria produce L-lysine in an improved manner after elimination of the mez gene.

The nucleotide sequence of the gene which codes for the malate enzyme of Corynebacterium glutamicum can be found in the patent application FR-A-2796080. The nucleotide sequence of the gene is deposited in the databank of the National Center for Biotechnology Information (NCBI) of the National Library of Medicine (Bethesda, MD, USA) under Accession Number AF234535.

The nucleotide sequence of the gene which codes for the malate enzyme of Corynebacterium glutamicum can furthermore be found in the patent application EP1108790 as sequence no. 3328 and as sequence no. 7069 and the patent application W00100844 from sequence no. 577 under Identification Code RXN10148. The nucleotide sequences are also deposited in the databank of the National Center for Biotechnology Information (NCBI) of the National Library of Medicine (Bethesda, MD, USA) under Accession Number AX123412, under Accession Number AX127153 and under Accession Number AX065451.

The patent application FR 2796080 discloses that by enhancement of the malate enzyme in Corynebacterium glutamicum, an increased production of amino acids, in particular lysine, is achieved.

Surprisingly, it has been found, in contrast to the prior art (FR 2796080), that coryneform bacteria produce L-lysine in an improved manner after elimination of the mez gene.

The sequence of the mez gene from Corynebacterium glutamicum ATCC13032, which codes for the malate enzyme, is shown in SEQ ID No. 1 and can be used according to the invention. The amino acid sequence of the protein is shown in SEQ ID no. 2.

The nucleotide sequence of the mez gene from Corynebacterium glutamicum shown in SEQ ID No. 1 differs from the nucleotide sequence described in the patent application FR 2796080 at positions 138, 186, 351, 477, 483, 603, 606, 609, 612, 624, 627, 654, 657, 666, 719, 831, 837, 846, 849, 850, 1014, 1029, 1052 and 1111. The nucleobase differences at position 719 and at position 850 of the nucleotide sequence lead to exchanges in the amino acid sequence, at position 240 of the amino acid sequence aspartate is present instead of alanine and at position 284 of the amino acid sequence glutamine is present instead of glutamate.

Alleles of the malate enzyme which result from the degeneracy of the genetic code or due to "sense mutations" of neutral function can furthermore be used.

To achieve an attenuation, either the expression of the mez gene or the catalytic properties of the gene products can be reduced or eliminated. The two measures are optionally combined.

The gene expression can be reduced by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1999)) and in known textbooks of genetics and molecular biology, such as e.g. the textbook by Knippers ("Molekulare Genetik", 6th edition, Georg Thieme Verlag, Stuttgart, Germany, 1995) or that by Winnacker ("Gene und Klone", VCH Verlagsgesellschaft, Weinheim, Germany, 1990).

A further method for specific reduction of gene expression is the antisense technique, in which short oligodeoxynucleotides or vectors for synthesis of longer antisense RNA are brought into the target cells. The antisense RNA can bond there to complementary sections of specific mRNAs and reduce the stability thereof or block the translatability. An example in this context is to be found by the expert in Srivastava et al. (Applied Environmental Microbiology 2000 Oct; 66 (10): 4366 - 4371).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms", Reports from the Jülich Research Center, Jül-2906, ISSN09442952, Jülich, Germany, 1994). Summarizing descriptions can be found in known textbooks of genetics and molecular biology, such as e.g. that by Hagemann ("Allgemeine Genetik", Gustav Fischer Verlag, Stuttgart, 1986).

Possible mutations are transitions, transversions, insertions and deletions of at least one base pair. Depending on the effect of the amino acid exchange on the enzyme activity, "missense mutations" or "nonsense mutations" are referred to. As a consequence of nonsense mutations sense codons are converted into stop codons and translation terminates prematurely. Insertions or deletions of at least one base pair in a gene lead to "frame shift mutations", as a consequence of which incorrect amino acids are incorporated or translation is interrupted prematurely. Deletions of one or more codons typically lead to a complete loss of the enzyme activity. Instructions on generation of such mutations are prior art and can be found in known textbooks of genetics and molecular biology, such as e.g. the textbook by Knippers ("Molekulare Genetik", 6th edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

A common method of mutating genes of C. glutamicum is the method of "gene disruption" and "gene replacement" described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 (1992)), pGEM-T (Promega Corporation, Madison, WI, USA), pCR2.1-TOPO (Shuman (1994). Journal of Biological Chemistry 269:32678-84; US Patent 5,487,993), pCR®Blunt (Invitrogen, Groningen, Holland; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of C. glutamicum.

In the method of "gene replacement", a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch et al. (Microbiology 144, 915 - 927 (1998)) to eliminate the pyc gene of C. glutamicum by a deletion.

A deletion, insertion or a base exchange can be incorporated into the mez gene in this manner.

In addition, it may be advantageous for the production of L-lysine to over-express, one or more enzymes of the particular biosynthesis pathway, of glycolysis, of anaplerosis, of the citric acid cycle, of the pentose phosphate cycle, of amino acid export and optionally regulatory proteins, in addition to the attenuation of the mez gene.

The term "enhancement" or "enhance" in this connection describes the increase in the intracellular activity or concentration of one or more enzymes or proteins in a microorganism which are coded by the corresponding DNA, for example by increasing the number of copies of the gene or genes, using a potent promoter or a gene or allele which codes for a corresponding enzyme or protein with a high activity, and optionally combining these measures.

By over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The use of endogenous genes is in general preferred. "Endogenous genes" or "endogenous nucleotide sequences" are understood as meaning the genes or nucleotide sequences present in the population of a species.

Thus, for the preparation of L-lysine, in addition to elimination of the mez gene one or more of the genes chosen from the group consisting of
- the lysC gene which codes for a feed-back resistant aspartate kinase (Accession No.P26512, EP-B-0387527; EP-A-0699759; WO 00/63388),
- the dapA gene which codes for dihydrodipicolinate synthase (EP-B 0 197 335),
- the gap gene which codes for glyceraldehyde 3-phosphate dehydrogenase (Eikmanns (1992). Journal of Bacteriology 174:6076-6086),
- at the same time the pyc gene which codes for pyruvate carboxylase (EP-A-1083225),
- the mqo gene which codes for malate:quinone oxidoreductase (Molenaar et al., European Journal of Biochemistry 254, 395 - 403 (1998); EP-A-1038969),
- the zwf gene which codes for glucose 6-phosphate dehydrogenase (JP-A-09224661, WO 01/70995),
- at the same time the lysE gene which codes for the lysine export protein (DE-A-195 48 222),
- the zwa1 gene which codes for the Zwa1 protein (EP-A-1111062)
- the tpi gene which codes for triose phosphate isomerase (Eikmanns (1992), Journal of Bacteriology 174:6076-6086), and
- the pgk gene which codes for 3-phosphoglycerate kinase (Eikmanns (1992), Journal of Bacteriology 174:6076-6086),
can be over-expressed.

It may furthermore be advantageous for the production of L-lysine, in addition to the elimination of the mez gene, at the same time for one or more of the genes chosen from the group consisting of
- the pck gene which codes for phosphoenol pyruvate carboxykinase (EP-A-1094111),
- the pgi gene which codes for glucose 6-phosphate isomerase (EP-A-1087015, WO 01/07626),
- the poxB gene which codes for pyruvate oxidase (EP-A-1096013),
- the zwa2 gene which codes for the Zwa2 protein (EP-A-1106693),
- the ccpA1 gene which codes for a catabolite control protein A (WO 02/18419),
to be eliminated.

Finally, in addition to elimination of the mez gene it may be advantageous for the production of amino acids to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The description also provides the microorganisms prepared for the claimed process and these can be cultured continuously or discontinuously in the batch process (batch culture) or in the fed batch (feed process) or repeated fed batch process (repetitive feed process) for the purpose of production of L-lysine. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen(Vieweg Verlag, Braunschweig/ Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of the desired product has formed. This target is usually reached within 10 hours to 160 hours.

Methods for the determination of L-lysine are known from the prior art. The analysis can thus be carried out as described by Spackman et al. (Analytical Chemistry, 30, (1958), 1190) by anion exchange chromatography with subsequent ninhydrin derivation, or it can be carried out by reversed phase HPLC, for example as described by Lindroth et al. (Analytical Chemistry (1979) 51: 1167-1174).

The present invention is explained in more detail in the following with the aid of embodiment examples.

### Example 1

### Deletion of the mez gene

For this, chromosomal DNA is isolated from the strain ATCC13032 by the method of Tauch et al. (1995, Plasmid 33:168-179). On the basis of the sequence of the mez gene known for C. glutamicum from the patent application EP1108790 as sequence no. 3328 and as sequence no. 7069, from the patent application W00100844 from sequence no. 577 under Identification Code RXN10148 and from the databank of the National Center for Biotechnology Information (NCBI) of the National Library of Medicine (Bethesda, MD, USA) under Accession Number AX123412, under Accession Number AX127153 and under Accession Number AX065451, the oligonucleotides described in the following are chosen for generation of the mez deletion allele by means of the polymerase chain reaction (PCR) by the "Splicing by Overlap Extension"-Method (Gene SOEing Method) (Horton, Molecular Biotechnology 3: 93-98 (1995)):
Primer mez_1 (see from SEQ ID No. 3):
   5'- ATG ACC ATC GAC CTG CAG CG -3'
Primer mez_2 (see from SEQ ID No. 4):
   5'- AAGAAGGCGCGATGGCTGCG -3'
Primer mez_3 (see from SEQ ID No. 5):
   5'-CGC AGC CAT CGC GCC TTC TTA ATG AGG CTT TCA CCG GCG C-3'
Primer mez_4 (see from SEQ ID No. 6):
   5'- AAG CGT TTT GCG CTT CGG CG -3'

The primers shown are synthesized by MWG Biotech (Ebersberg, Germany) and the PCR reaction is carried out using Pfu polymerase (Stratagene, Product. No. 600135, La Jolla, USA) and a PTC 100 Thermocycler (MJ Research Inc., Waltham, USA).

The primer mez_3 is composed of two regions of the nucleotide sequence which bond to nucleotides 384 to 403 and 766 to 785 within the coding sequence of mez.

With the aid of the polymerase chain reaction, the primers mez_1 and mez_2 allow amplification of a DNA fragment 403 bp in size and the primers mez_3 and mez_4 allow amplification of a DNA fragment 432 bp in size. The amplification products are tested electrophoretically in a 0.8% agarose gel, isolated from the agarose gel with the High Pure PCR Product Purification Kit (Product No. 1732676, Roche Diagnostics GmbH, Mannheim, Germany) and employed together as templates for a further PCR reaction with the primers mez_1 and mez_4. The mez deletion derivative 815 bp in size is generated in this manner (see also SEQ ID No. 7).

The product amplified in this way is tested electrophoretically in a 0.8% agarose gel.

### Example 2

### Cloning of the mez deletion derivative in the vector pCRBluntII

The amplified DNA fragment of 815 bp length which carries the mez deletion derivative is ligated with the Zero Blunt^{™} Kit of Invitrogen Corporation (Carlsbad, CA, USA; Catalogue Number K2700-20) in the vector pCR®Blunt II (Bernard et al., Journal of Molecular Biology, 234: 534-541 (1993)). The E. coli strain Top10 (Grant et al., Proceedings of the National Academy of Sciences USA, 87 (1990) 4645-4649) is then transformed with the ligation batch in accordance with the instructions of the manufacturer of the kit (Invitrogen Corporation, Carlsbad, CA, USA). Selection for plasmid-carrying cells is made by plating out the transformation batch on LB agar (Sambrook et al., Molecular Cloning: A Laboratory Manual. 2^{nd} Ed., Cold Spring Harbor Laboratory Press, Cold Spring Harbor, N.Y., 1989), which had been supplemented with 25 mg/l kanamycin. Plasmid DNA is isolated from a transformant with the aid of the QIAprep Spin Miniprep Kit from Qiagen (Hilden, Germany) and checked by treatment with the restriction enzymes EcoRV and EcoRI with subsequent agarose gel electrophoresis (0.8 %). The plasmid is called pCRBlunt_delmez.

### Example 3

### Construction of the exchange vector pK18mobsacBdeltamez

The mez deletion derivative is isolated from the plasmid pCRBlunt_delmez described in example 2 by complete cleavage with the enzyme EcoRI. After separation in an agarose gel (0.8%) with the High Pure PCR Product Purification Kit (Product No. 1732676, Roche Diagnostics GmbH, Mannheim, Germany), the fragment approx. 0.83 kb in size carrying the mez deletion derivative is isolated from the agarose gel.

The mez deletion derivative obtained in this way is employed for ligation with the mobilizable cloning vector pK18mobsacB (Schäfer et al. (1994), Gene 14: 69-73). This was cleaved completely beforehand with the restriction endonuclease EcoRI and subsequently dephosphorylated with shrimp alkaline phosphatase (Roche Diagnostics GmbH, Mannheim, Germany, Product Description SAP, Product No. 1758250). The vector prepared in this way is mixed with the mez deletion allele and the mixture is treated with T4 DNA ligase (Amersham-Pharmacia, Freiburg, Germany).

The E. coli strain DH5αmcr (Grant, 1990, Proceedings of the National Academy of Sciences U.S.A., 87:4645-4649) is then electroporated with the ligation batch (Hanahan, In. DNA Cloning. A Practical Approach, Vol. 1, ILR-Press, Cold Spring Habor, New York, 1989). Selection of plasmid-carrying cells is made by plating out the transformation batch on LB agar(Sambrook et al., Molecular Cloning: A Laboratory Manual. 2^{nd} Ed., Cold Spring Harbor, New York, 1989), which has been supplemented with 25 mg/l kanamycin.

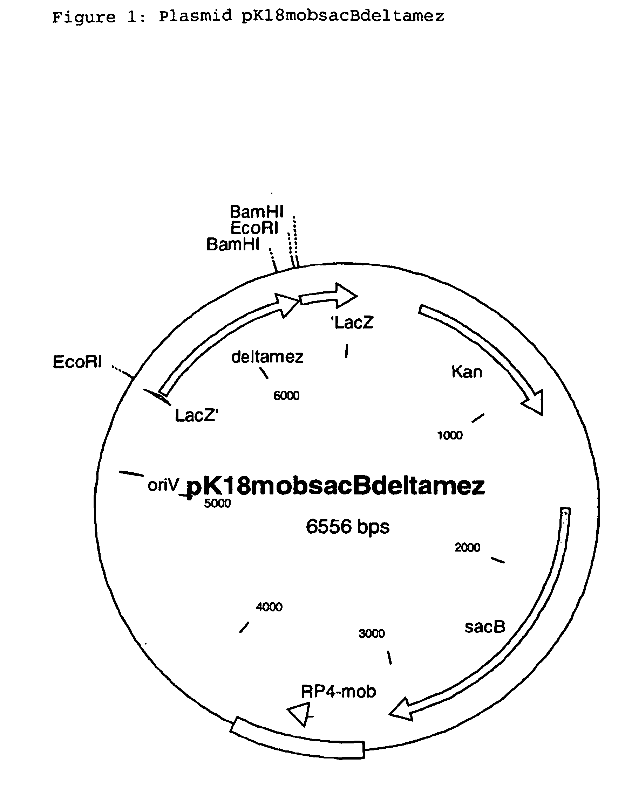

Plasmid DNA is isolated from a transformant with the aid of the QIAprep Spin Miniprep Kit from Qiagen and the cloned mez deletion allele is verified by means of restriction cleavage with the restriction endonucleases EcoRI and BamHI. The plasmid is called pK18mobsacBdeltamez and is shown in figure 1. The strain is called E.coliDH5αmcr/pK18mobsacBdeltamez.

### Example 4

### Deletion mutagenesis of the mez gene in the C. glutamicum strain DM1637

The Corynebacterium glutamicum strain DM1637 was prepared by multiple, non-directed mutagenesis, selection and mutant selection from Corynebacterium glutamicum ATCC21527. The strain is resistant to the lysine analogue S-(2-aminoethyl)-L-cysteine and auxotrophic for the amino acids L-methionine and L-threonine.

The vector pK18mobsacBdeltamez mentioned in example 3 is transferred by means of conjugation by a protocol of Schafer et al. (Journal of Microbiology 172: 1663-1666) (1990)) into the Corynebacterium glutamicum strain DM1637. The vector cannot replicate independently in DM1637 and is retained in the cell only if it has integrated into the chromosome as the consequence of a recombination event. Selection of clones with integrated pK18mobsacBdeltamez is carried out by plating out the conjugation batch on LB agar (Sambrook et al., Molecular Cloning: A Laboratory Manual, 2^{nd} Ed., Cold Spring Habor, New York, 1989), which has been supplemented with 15 mg/l kanamycin and 50 mg/ml nalidixic acid. Clones which have grown on are plated out on LB agar plates with 25 mg/l kanamycin and incubated for 16 hours at 33°C. For selection of mutants in which excision of the plasmid has taken place as a consequence of a second recombination event, the clones are cultured unselectively for 20 hours in LB liquid medium and then plated out on LB agar with 10% sucrose and incubated for 24 hours.

The plasmid pK18mobsacBdeltamez, like the starting plasmid pK18mobsacB, contains, in addition to the kanamycin resistance gene, a copy of the sacB gene which codes for levan sucrase from Bacillus subtilis. The expression which can be induced by sucrose leads to the formation of levan sucrase, which catalyses the synthesis of the product levan, which is toxic to C. glutamicum. Only those clones in which the pK18mobsacBdeltamez integrated has been excised again therefore grow on LB agar with sucrose. In the excision, together with the plasmid either the complete chromosomal copy of the mez gene can be excised, or the incomplete copy with the internal deletion.

Approximately 40 to 50 colonies are tested for the phenotype "growth in the presence of sucrose" and "non-growth in the presence of kanamycin". To demonstrate that deleted mez allele has remained in the chromosome, approximately 20 colonies which show the phenotype "growth in the presence of sucrose" and "non-growth in the presence of kanamycin" are investigated with the aid of the polymerase chain reaction by the standard PCR method of Innis et al. (PCR protocols. A Guide to Methods and Applications, 1990, Academic Press). A DNA fragment which carries the mez gene and surrounding regions is amplified here from the chromosomal DNA of the colonies. The following primer oligonucleotides are chosen for the PCR.
mez-A1 (SEQ ID NO: 8):
   5' agt agc agc cca aat tca gc 3'
mez-E1 (SEQ ID NO: 9):
   5' ggg cct caa gtt tgc tct ta 3'

The primers allow amplification of a DNA fragment approx. 1.5 kb in size in control clones with the complete mez allele. In clones with a deleted mez allele, DNA fragments with a size of approx. 1.14 kb are amplified.

The amplified DNA fragments are identified by means of electrophoresis in a 0.8% agarose gel. It could thus be demonstrated that the strain DM1637 carries a deleted mez allele on the chromosome. The strain was called C. glutamicum DM1637deltamez.

### Example 5

### Preparation of lysine

The C. glutamicum strain DM1637deltamez obtained in example 4 is cultured in a nutrient medium suitable for the production of lysine and the lysine content in the culture supernatant is determined.

For this, the strain is first incubated on an agar plate for 24 hours at 33 °C. Starting from this agar plate culture, a preculture is seeded (10 ml medium in a 100 ml conical flask). The medium MM is used as the medium for the preculture. The preculture is incubated for 24 hours at 33ºC at 240 rpm on a shaking machine. A main culture is seeded from this preculture such that the initial OD (660 nm) of the main culture is 0.1. The Medium MM is also used for the main culture.

| Medium MM | |
|---|---|
| CSL | 5 g/l |
| MOPS | 20 g/l |
| Glucose (autoclaved separately) | 50 g/l |
| Salts: | |
| (NH₄)₂SO₄ | 25 g/l |
| KH₂PO₄ | 0.1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Homoserine (sterile-filtered) | 0.4 g/l |
| CaCO₃ | 25 g/l |

The CSL (corn steep liquor), MOPS (morpholinopropanesulfonic acid) and the salt solution are brought to pH 7 with aqueous ammonia and autoclaved. The sterile substrate and vitamin solutions, as well as the CaCO₃ autoclaved in the dry state, are then added.

Culturing is carried out in a 10 ml volume in a 100 ml conical flask with baffles. Culturing is carried out at 33ºC and 80% atmospheric humidity.

After 48 hours, the OD is determined at a measurement wavelength of 660 nm with a Biomek 1000 (Beckmann Instruments GmbH, Munich). The amount of lysine formed is determined with an amino acid analyzer from Eppendorf-BioTronik (Hamburg, Germany) by ion exchange chromatography and post-column derivation with ninhydrin detection.

The result of the experiment is shown in Table 1.

**Table 1**

| Strain | OD (660 nm) | Lysine HCl g/l |
|---|---|---|
| DM1637 | 10.9 | 11.3 |
| DM1637deltamez | 10.8 | 11.8 |

### Brief Description of the Figure:

Figure 1: Map of the plasmid pK18mobsacBdeltamez

The base pair numbers stated are approximate values obtained in the context of reproducibility of measurements.

The abbreviations and designations used have the following meaning:

| | |
|---|---|
| oriV: | ColEl-similar origin from PMB1 |
| sacB | the sacB gene which codes for the protein levan sucrase |
| RP4mob: | RP4 mobilization site |
| Km: | resistance gene for kanamycin |
| LacZ' : | 5'-terminus of the lacZα gene fragment |
| 'LacZ: | 3'-terminus of the lacZα gene fragment |
| deltamez: | deleted allele of the mez gene from C. glutamicum |
| BamHI: | cleavage site of the restriction enzyme BamHI |
| EcoRI: | cleavage site of the restriction enzyme EcoRI |

## Claims

1. A process for the preparation of L-lysine by fermentation of a recombinant coryneform bacteria, wherein in the bacteria the expression of the nucleotide sequence of Corynebacterium glutamicum which codes for the malate enzyme is eliminated.

2. Process according to claim 1, further comprising:
a) concentration of the L-lysine in the medium or in the cells of the bacteria, and
b) isolation of the L-lysine, constituents of the fermentation broth and/or the biomass optionally remaining in portions or in their total amounts in the end product.

3. Process according to claim 1 or 32, wherein coryneform microorganisms are fermented in which at the same time one or more of the genes chosen from the group consisting of
3.1 the lysC gene which codes for a feed-back resistant aspartate kinase,
3.2 the dapA gene which codes for dihydrodipicolinate synthase,
3.3 the gap gene which codes for glyceraldehyde 3-phosphate dehydrogenase,
3.4 the pyc gene which codes for pyruvate carboxylase,
3.5 the mqo gene which codes for malate:quinone oxidoreductase,
3.6 the zwf gene which codes for glucose 6-phosphate dehydrogenase,
3.7 at the same time the lysE gene which codes for the lysine export protein,
3.8 the zwa1 gene which codes for the Zwa1 protein,
3.9 the tpi gene which codes for triose phosphate isomerase, and
3.10 the pgk gene which codes for 3-phosphoglycerate kinase,
is or are over-expressed.

4. Process according to claim 1 or 2, wherein coryneform microorganisms are fermented in which at the same time one or more of the genes chosen from the group consisting of
9.1 the pck gene which codes for phosphoenol pyruvate carboxykinase,
9.2 the pgi gene which codes for glucose 6-phosphate isomerase,
9.3 the poxB gene which codes for pyruvate oxidase,
4.4 the zwa2 gene which codes for the Zwa2 protein, and
4.5 the ccpA1 gene which codes for a catabolite control protein A,
is or are eliminated.

5. Process according to one or more of the preceding claims, wherein microorganisms of the species Corynebacterium glutamicum are employed.

## Patentansprüche

1. Verfahren zur Herstellung von L-Lysin durch Fermentation rekombinanter coryneformer Bakterien, bei dem man in den Bakterien die Exprimierung der für das Malat-Enzym kodierenden Nukleotidsequenz von Corynebacterium glutamicum ausschaltet.

2. Verfahren nach Anspruch 1, bei dem man ferner
a) das L-Lysin im Medium oder in den Zellen der Bakterien anreichert und
b) das L-Lysin isoliert, wobei gegebenenfalls Bestandteile der Fermentationsbrühe und/oder der Biomasse in Anteilen oder ihren Gesamtmengen im Endprodukt verbleiben.

3. Verfahren nach Anspruch 1 oder 2, bei dem man coryneforme Mikroorganismen fermentiert, indem man gleichzeitig eines oder mehrere der Gene, ausgewählt aus der Gruppe:
3.1 das für eine feed-back resistente Aspartatkinase kodierende Gen lysC,
3.2 das für die Dihydrodipicolinat-Synthase kodierende Gen dapA,
3.3 das für die Glyceraldehyd-3-Phosphat-Dehydrogenase kodierende Gen gap,
3.4 das für die Pyruvat-Carboxylase kodierende Gen pyc,
3.5 das für die Malat:Chinon-Oxidoreduktase kodierende Gen mqo,
3.6 das für die Glucose-6-Phosphat-Dehydrogenase kodierende Gen zwf,
3.7 gleichzeitig das für das Lysin-Export-Protein kodierende Gen lysE,
3.8 das für das Zwa1-Protein kodierende Gen zwa1,
3.9 das für die Triosephosphat-Isomerase kodierende Gen tpi und
3.10 das für die 3-Phosphoglycerat-Kinase kodierende Gen pgk,
überexprimiert.

4. Verfahren nach Anspruch 1 oder 2, bei dem man coryneforme Mikroorganismen fermentiert, in denen man gleichzeitig eines oder mehrere der Gene, ausgewählt aus der Gruppe:
4.1 das für die Phosphoenolpyruvat-Carboxykinase kodierende pck-Gen,
4.2 das für die Glucose-6-Phosphat-Isomerase kodierende pgi-Gen,
4.3 das für die Pyruvat-Oxidase kodierende Gen poxB,
4.4 das für das Zwa2-Protein kodierende Gen zwa2, oder
4.5 das für ein Katabolit-Kontroll-Protein A kodierende Gen ccpA1,
ausschaltet.

5. Verfahren nach einem oder mehreren der vorhergehenden Ansprüche, bei dem man Mikroorganismen der Art Corynebacterium glutamicum einsetzt.

## Revendications

1. Procédé pour la préparation de L-lysine par fermentation d'une bactérie coryneforme recombinante, dans lequel on a éliminé, dans la bactérie, l'expression de la séquence nucléotidique de l'espèce Corynebacterium glutamicum qui code pour l'enzyme du malate.

2. Procédé selon la revendication 1, comprenant en outre :
a) la concentration de la L-lysine dans le milieu ou dans les cellules bactériennes, et
b) l'isolement de la L-lysine, de constituants du bouillon de fermentation et/ou de la biomasse, qui restent éventuellement sous la forme de portions ou de leurs quantités totales dans le produit final.

3. Procédé selon la revendication 1 ou 2, dans lequel on fait fermenter des microorganismes coryneformes dans lesquels est effectuée, en même temps, la surexpression d'un ou de plusieurs gènes choisis dans le groupe constitué :
3.1 du gène lysC qui code pour une aspartate-kinase résistant à la rétroaction,
3.2 du gène dapA qui code pour la dihydrodipicolinate-synthase,
3.3 du gène gap qui code pour la glycéraldéhyde-3-phosphate déshydrogénase,
3.4 du gène pyc qui code pour la pyruvate-carboxylase,
3.5 du gène mqo qui code pour la malate:quinone-oxydoréductase,
3.6 du gène zwf qui code pour la glucose-6-phosphate déshydrogénase,
3.7 en même temps gène lysE qui code pour la protéine d'export de la lysine,
3.8 du gène zwa1 qui code pour la protéine Zwa1,
3.9 du gène tpi qui code pour la triosephosphate-isomérase, et
3.10 du gène pgk qui code pour la 3-phospho-glycérate-kinase.

4. Procédé selon la revendication 1 ou 2, dans lequel on fait fermenter des microorganismes coryneformes dans lesquels on a éliminé, en même temps, un ou plusieurs gènes choisis dans le groupe constitué :
4.1 du gène pck qui code pour la phosphoénolpyruvate-carboxykinase,
4.2 du gène pgi qui code pour la glucose-6-phosphate isomérase,
4.3 du gène poxB qui code pour la pyruvate-oxydase,
4.4 du gène zwa2 qui code pour la protéine Zwa2, et
4.5 du gène ccpA1 qui code pour une protéine A de contrôle de catabolites.

5. Procédé selon l'une ou plusieurs des revendications précédentes, dans lequel on utilise des microorganismes de l'espèce Corynebacterium glutamicum.


### SEQUENCE LISTING

<110> Degussa AG
<120> Process for the preparation of L-lysine using coryneform bacteria which contain an attenuated mez gene
<130> 020175 BT
<160> 9
<170> PatentIn Version 3.1
<210> 1
   <211> 1179
   <212> DNA
   <213> Corynebacterium glutamicum
<220>
   <221> CDS
   <222> (1)..(1176)
   <223> mez gene
<400> 1
   atg acc atc gac ctg cag cgt tcc acc caa aac ctc acc cat gag gaa      48
   Met Thr Ile Asp Leu Gln Arg Ser Thr Gln Asn Leu Thr His Glu Glu
   1               5                   10                  15
   atc ttc gag gca cac gag ggc gga aag ctc tcc att agt tcc act cgt      96
   Ile Phe Glu Ala His Glu Gly Gly Lys Leu Ser Ile Ser Ser Thr Arg
               20                  25                  30
   ccg ctc cgc gac atg cgc gat ctt tcc ctt gct tac acc cct ggt gtt     144
   Pro Leu Arg Asp Met Arg Asp Leu Ser Leu Ala Tyr Thr Pro Gly Val
           35                  40                  45
   gct cag gtt tgt gaa gca atc aag gaa gat cca gag gtt gcg cgc acc     192
   Ala Gln Val Cys Glu Ala Ile Lys Glu Asp Pro Glu Val Ala Arg Thr
       50                  55                  60
   cac acg ggc att gga aac acc gtc gcg gtt att tcc gac ggc acc gct     240
   His Thr Gly Ile Gly Asn Thr Val Ala Val Ile Ser Asp Gly Thr Ala
   65                  70                  75                  80
   gtt ctt ggc ctt ggc gat atc gga cct cag gcc tcc ctt ccc gtc atg     288
   Val Leu Gly Leu Gly Asp Ile Gly Pro Gln Ala Ser Leu Pro Val Met
                   85                  90                  95
   gag ggc aag gct cag ctg ttt agc tct ttc gct ggc ctg aag gct atc     336
   Glu Gly Lys Ala Gln Leu Phe Ser Ser Phe Ala Gly Leu Lys Ala Ile
               100                 105                 110
   cct atc gtt ttg gac gtt cac gat gtt gac gct ttg gtt gag acc atc     384
   Pro Ile Val Leu Asp Val His Asp Val Asp Ala Leu Val Glu Thr Ile
           115                 120                 125
   gca gcc atc gcg cct tct ttc ggt gct atc aac ttg gag gac atc tcc      432
   Ala Ala Ile Ala Pro Ser Phe Gly Ala Ile Asn Leu Glu Asp Ile Ser
       130                 135                 140
   gct cct cgt tgc ttc gag gtg gag cgc cgc ctc atc gag cgt ctc gat      480
   Ala Pro Arg Cys Phe Glu Val Glu Arg Arg Leu Ile Glu Arg Leu Asp
   145                 150                 155                 160
   att cca gtt atg cac gat gac cag cac ggc acc gct gtg gtt atc ctc      528
   Ile Pro Val Met His Asp Asp Gln His Gly Thr Ala Val Val Ile Leu
                   165                 170                 175
   gct gcg ctg cgc aac tcc ctg aag ctg ctg gat cgc aag atc gaa gac      576
   Ala Ala Leu Arg Asn Ser Leu Lys Leu Leu Asp Arg Lys Ile Glu Asp
               180                 185                 190
   ctc aag att gtt att tcc ggc gca ggc gca gcg ggc gtt gca gct gta      624
   Leu Lys Ile Val Ile Ser Gly Ala Gly Ala Ala Gly Val Ala Ala Val
           195                 200                 205
   gat atg ctg acc aac gct gga gca acc gac atc gtg gtt ctt gat tcc      672
   Asp Met Leu Thr Asn Ala Gly Ala Thr Asp Ile Val Val Leu Asp Ser
       210                 215                 220
   cga ggc atc atc cac gac agc cgt gag gat ctt tcc cca gtt aag gct      720
   Arg Gly Ile Ile His Asp Ser Arg Glu Asp Leu Ser Pro Val Lys Ala
   225                 230                 235                 240
   gct ctt gca gag aag acc aac cct cgt ggc atc agc ggt ggc atc aat      768
   Ala Leu Ala Glu Lys Thr Asn Pro Arg Gly Ile Ser Gly Gly Ile Asn
                   245                 250                 255
   gag gct ttc acc ggc gcg gac ctg ttc att ggc gtg tcc ggc ggc aac      816
   Glu Ala Phe Thr Gly Ala Asp Leu Phe Ile Gly Val Ser Gly Gly Asn
               260                 265                 270
   atc ggc gag gac gct ctc aaa ctc atg gcc ccg gag cca atc ctg ttc      864
   Ile Gly Glu Asp Ala Leu Lys Leu Met Ala Pro Glu Pro Ile Leu Phe
           275                 280                 285
   acc ctg gcg aac cca acc cca gag atc gat cct gag ctg tct cag aag      912
   Thr Leu Ala Asn Pro Thr Pro Glu Ile Asp Pro Glu Leu Ser Gln Lys
       290                 295                 300
   tac ggc gcc atc gtc gcg acc ggc cgc tct gac ctg cct aac cag atc      960
   Tyr Gly Ala Ile Val Ala Thr Gly Arg Ser Asp Leu Pro Asn Gln Ile
   305                 310                 315                 320
   aac aac gtg ctc gcg ttc cca gga att ttc gcc ggc gct ctc gca gcc     1008
   Asn Asn Val Leu Ala Phe Pro Gly Ile Phe Ala Gly Ala Leu Ala Ala
                   325                 330                 335
   aag gct aag aag atc acc ccc gag atg aag ctc gcc gct gca gag gca     1056
   Lys Ala Lys Lys Ile Thr Pro Glu Met Lys Leu Ala Ala Ala Glu Ala
               340                 345                 350
   atc gcc gac atc gca gct gag gac ctc gag gtc ggc cgc atc gtg cct     1104
   Ile Ala Asp Ile Ala Ala Glu Asp Leu Glu Val Gly Arg Ile Val Pro
           355                 360                 365
   acc gcc ctg gat ccc cgc gtc gcc cca gca gtc aag gca gct gtc cag    1152
   Thr Ala Leu Asp Pro Arg Val Ala Pro Ala Val Lys Ala Ala Val Gln
       370                 375                     380
   gcc gtc gcc gaa gcg caa aac gct taa                                1179
   Ala Val Ala Glu Ala Gln Asn Ala
   385                 390
<210> 2
   <211> 392
   <212> PRT
   <213> Corynebacterium glutamicum
<400> 2
   Met Thr Ile Asp Leu Gln Arg Ser Thr Gln Asn Leu Thr His Glu Glu
   1 5 10 15
   Ile Phe Glu Ala His Glu Gly Gly Lys Leu Ser Ile Ser Ser Thr Arg
   20 25 30
   Pro Leu Arg Asp Met Arg Asp Leu Ser Leu Ala Tyr Thr Pro Gly Val
   35 40 45
   Ala Gln Val Cys Glu Ala Ile Lys Glu Asp Pro Glu Val Ala Arg Thr
   50 55 60
   His Thr Gly Ile Gly Asn Thr Val Ala Val Ile Ser Asp Gly Thr Ala
   65 70 75 80
   Val Leu Gly Leu Gly Asp Ile Gly Pro Gln Ala Ser Leu Pro Val Met
   85 90 95
   Glu Gly Lys Ala Gln Leu Phe Ser Ser Phe Ala Gly Leu Lys Ala Ile
   100 105 110
   Pro Ile Val Leu Asp Val His Asp Val Asp Ala Leu Val Glu Thr Ile
   115 120 125
   Ala Ala Ile Ala Pro Ser Phe Gly Ala Ile Asn Leu Glu Asp Ile Ser
   130 135 140
   Ala Pro Arg Cys Phe Glu Val Glu Arg Arg Leu Ile Glu Arg Leu Asp
   145 150 155 160
   Ile Pro Val Met His Asp Asp Gln His Gly Thr Ala Val Val Ile Leu
   165 170 175
   Ala Ala Leu Arg Asn Ser Leu Lys Leu Leu Asp Arg Lys Ile Glu Asp
   180 185 190
   Leu Lys Ile Val Ile Ser Gly Ala Gly Ala Ala Gly Val Ala Ala Val
   195 200 205
   Asp Met Leu Thr Asn Ala Gly Ala Thr Asp Ile Val Val Leu Asp Ser
   210 215 220
   Arg Gly Ile Ile His Asp Ser Arg Glu Asp Leu Ser Pro Val Lys Ala
   225 230 235 240
   Ala Leu Ala Glu Lys Thr Asn Pro Arg Gly Ile Ser Gly Gly Ile Asn
                   245                 250                 255
   Glu Ala Phe Thr Gly Ala Asp Leu Phe Ile Gly Val Ser Gly Gly Asn
               260                 265                 270
   Ile Gly Glu Asp Ala Leu Lys Leu Met Ala Pro Glu Pro Ile Leu Phe
           275                 280                 285
   Thr Leu Ala Asn Pro Thr Pro Glu Ile Asp Pro Glu Leu Ser Gln Lys
       290                 295                 300
   Tyr Gly Ala Ile Val Ala Thr Gly Arg Ser Asp Leu Pro Asn Gln Ile
   305                 310                 315                 320
   Asn Asn Val Leu Ala Phe Pro Gly Ile Phe Ala Gly Ala Leu Ala Ala
                   325                 330                 335
   Lys Ala Lys Lys Ile Thr Pro Glu Met Lys Leu Ala Ala Ala Glu Ala
               340                 345                 350
   Ile Ala Asp Ile Ala Ala Glu Asp Leu Glu Val Gly Arg Ile Val Pro
           355                 360                 365
   Thr Ala Leu Asp Pro Arg Val Ala Pro Ala Val Lys Ala Ala Val Gln
       370                 375                 380
   Ala Val Ala Glu Ala Gln Asn Ala
   385                 390
<210> 3
   <211> 20
   <212> DNA
   <213> Corynebacterium glutamicum
<220>
   <221> misc_feature
   <222> (1)..(20)
   <223> Primer mez_1
<400> 3
   atgaccatcg acctgcagcg 20
<210> 4
   <211> 20
   <212> DNA
   <213> Corynebacterium glutamicum
<220>
   <221> misc_feature
   <222> (1)..(20)
   <223> Primer mez_2
<400> 4
   aagaaggcgc gatggctgcg 20
<210> 5
   <211> 40
   <212> DNA
   <213> Artificial sequence
<220>
   <221> misc_feature
   <222> (1)..(40)
   <223> Primer mez_3
<400> 5
   cgcagccatc gcgccttctt aatgaggctt tcaccggcgc 40
<210> 6.
   <211> 20
   <212> DNA
   <213> Corynebacterium glutamicum
<220>
   <221> misc_feature
   <222> (1)..(20)
   <223> Primer mez_4
<400> 6
   aagcgttttg cgcttcggcg 20
<210> 7
   <211> 815
   <212> DNA
   <213> Artificial sequence
<220>
   <221> misc_feature
   <222> (1)..(815)
   <223> PCR product: mez deletion derivative
<400> 7
   atgaccatcg acctgcagcg ttccacccaa aacctcaccc atgaggaaat cttcgaggca 60
   cacgagggcg gaaagctctc cattagttcc actcgtccgc tccgcgacat gcgcgatctt 120
   tcccttgctt acacccctgg tgttgctcag gtttgtgaag caatcaagga agatccagag 180
   gttgcgcgca cccacacggg cattggaaac accgtcgcgg ttatttccga cggcaccgct 240
   gttcttggcc ttggcgatat cggacctcag gcctcccttc ccgtcatgga gggcaaggct 300
   cagctgttta gctctttcgc tggcctgaag gctatcccta tcgttttgga cgttcacgat 360
   gttgacgctt tggttgagac catcgcagcc atcgcgcctt cttaatgagg ctttcaccgg 420
   cgcggacctg ttcattggcg tgtccggcgg caacatcggc gaggacgctc tcaaactcat 480
   ggccccggag ccaatcctgt tcaccctggc gaacccaacc ccagagatcg atcctgagct 540
   gtctcagaag tacggcgcca tcgtcgcgac cggccgctct gacctgccta accagatcaa 600
   caacgtgctc gcgttcccag gaattttcgc cggcgctctc gcagccaagg ctaagaagat 660
   cacccccgag atgaagctcg ccgctgcaga ggcaatcgcc gacatcgcag ctgaggacct 720
   cgaggtcggc cgcatcgtgc ctaccgccct ggatccccgc gtcgccccag cagtcaaggc 780
   agctgtccag gccgtcgccg aagcgcaaaa cgctt 815
<210> 8
   <211> 20
   <212> DNA
   <213> Corynebacterium glutamicum
<220>
   <221> misc_feature
   <222> (1)..(20)
   <223> Primer mez-A1
<400> 8
   agtagcagcc caaattcagc 20
<210> 9
   <211> 20
   <212> DNA
   <213> Corynebacterium glutamicum
<220>
   <221> misc_feature
   <222> (1)..(20)
   <223> Primer mez-E1
<400> 9
   gggcctcaag tttgctctta 20